(19) Europäisches Patentamt
European Patent Office
Office européen des brevets

(11) **EP 3 570 015 A1**

(12) **EUROPEAN PATENT APPLICATION**

(43) Date of publication:
**20.11.2019 Bulletin 2019/47**

(51) Int Cl.:
*G01N 23/041* (2018.01)     *A61B 6/00* (2006.01)

(21) Application number: **18173143.1**

(22) Date of filing: **18.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **AGFA NV**
**2640 Mortsel (BE)**

(72) Inventors:
• **NEBOSIS, Rainer**
  **D-81539 München (DE)**
• **VANDENBROUCKE, Dirk**
  **D-81539 München (DE)**

(54) **ALIGNMENT METHOD FOR PCI GRATING**

(57)     This invention is related to a method for alignment optimization of a pre-sample mask in a coded aperture PCI system based on the acquisition of an adjustment image which is analysed to calculate and perform the corrections of the different positional parameters of the pre-sample mask.

Fig. 1

## Description

### Technical Field

This invention is related to coded aperture phase contrast imaging, and more particularly to a method to compensate for alignment inaccuracies between the pre-sample mask and the detector grating. The compensation that is defined in terms of adjustment of the magnification factor (ratio of source - detector distance and source pre-sample mask distance), the adjustment of the lateral displacement of the pre-sample mask or the adjustment of the angle between the pre-sample mask and the detector grating, and is calculated in this method based on the acquisition and analysis of a single adjustment image acquired on the non-calibrated PCI system. The compensation may similarly be effectuated by providing artefact compensation during back-calculation of the respective images (refraction, scatter and absorption) based on the calculated deviations from the optimal positioning.

### Background of the invention

Phase-contrast X-ray imaging (PCI) is a general term for different technical methods that use information concerning changes in the phase of an X-ray beam that passes through an object in order to create its images. Standard X-ray imaging techniques like radiography or computed tomography (CT) rely on a decrease of the X-ray beam's intensity (attenuation) when traversing the sample, which can be measured directly with the assistance of an X-ray detector. In PCI however, the beam's phase shift and scattering caused by the sample is not measured directly, but is transformed into variations in intensity, which then can be recorded by the detector.

Conventional X-ray imaging uses the drop in intensity through attenuation caused by an object in the X-ray beam and the radiation is treated as rays like in geometrical optics. But when X-rays pass through an object, not only their amplitude but their phase and propagation direction is altered as well. When applying the values of typical human tissue, the phase-shift of an X-ray beam propagating through tissue is up to three orders of magnitude larger than the loss in intensity caused by the absorption by the same tissue, thus making PCI more sensitive to density variations in soft tissue than absorption imaging.

The present invention is applicable to the phase contrast imaging technique known as Coded Aperture Phase Contrast Imaging. The principle of coded aperture phase contrast imaging has been described in US 7,869,567.

X-rays emitted from an X-ray source are shaped into individual beamlets by means of selective absorption by a so-called "pre-sample mask" or "coded aperture" (which is an absorbing grating). The pre-sample mask allows only narrow portions of the X-ray beam to pass through so-called apertures typically of identical width. The pre-sample mask traditionally has a fixed geometry that is adapted to a detector grating or analyser grating that in its turn is fitted to the geometry of the used X-ray detector pixel grid dimensions while taking into account the distances between the X-ray source, pre-sample mask and X-ray detector.

These narrow beamlets pass through a sample or object and arrive at individual pixels of the X-ray detector through said detector grating. The individual X-ray beamlets are arranged to hit the pixel edge of individual rows of pixels or individual columns of pixels or individual pixels. Small deviations in the individual beamlets cause a significant increase or decrease in the signal hitting the exposed area of the pixel resulting in a significant phase contrast or dark field signal.

In order to perform phase contrast imaging successfully, very stringent requirements have to be met relating to accurate positioning of the different components in the system, as well as the very accurate construction and dimensioning of the essential parts (such as the pre-sample mask and the detector grating). In current PCI system embodiments, a pre-sampling mask with fixed geometry and an analyser grating that is fitted to the geometry of the used X-ray detector and/or the intensity distribution of the X-ray field at the detector is used. The different components are thus tailored to each other in order to meet the requirements for PCI. The required accuracy for positioning the different components in correct relation with each other are in the order of magnitude of $\mu$ms, and thus cannot be simply implemented by means of the available positioning means present in a conventional X-ray system. Special mechanical adaptation will be required. A calibration step followed by a number of adjustment steps will always be required in order to produce good quality PCI images.

For state of the art conventional X-ray systems, a direct implementation of PCI functionality is impossible. The main reason is that in principle, PCI requires a special X-ray tube (due to the period of the pre-sampling mask) with a very small focus or a special tube mask very close to the focal spot to produce spatial coherence (Talbot Lau approach), unless other measures are taken (such as the adoption of a virtual detector grating). A typical X-ray source in an X-ray modality first has a much larger focal spot than required for PCI with a standard pre-sampling mask.

Secondly the tube is fixed to a collimator which would not allow to insert a grating close to the tube exit window. The major requirement for the pre-sample mask adapter or holder is that it should allow very accurate adjustment of the positioning of the pre-sample mask.

Last but not least, the required positioning accuracy is extremely demanding, thus sharing of components is almost impossible for state of the art approaches.

Another limiting aspect of a conventional X-ray system is the X-ray detector. PCI requires an analyser grating that is positioned right in front of the detector. This grating must be perfectly aligned to the detector pixels. Adding and removing such a grating to/from the existing detector is technically feasible but also very demanding and requires high accuracy alignment. Moreover, it is advantageous for PCI that the digital image detector has to be positioned at a farther distance than is conventional (i.e. at a SDD of more than 120cm preferable more than 160cm). This latter aspect may be achieved by providing a dedicated detector holder.

In the state of the art, no suitable methods have been described that allow a fast and reliable optimization of the geometric setup of the different components of a PCI system, apart from laborious trial and error. For instance, US 9,171,650 describes a method to align the gratings of a PCI system with pre-sampling masks; the method requires to move and rotate the gratings to several positions/angles and find a maximum/minimum.

This invention intends to overcome these difficulties by providing a calibration method based on a single adjustment image acquisition. By consequence, this calibration technique offers the opportunity to use conventional X-ray systems in the application of PCI by foreseeing a limited number of technically feasible adaptations.

## Summary of invention

The present invention provides a method for calculating adjustments to the geometry of a PCI system. More specifically, it provides a method for calculating the discrepancy between 3 adjustable parameters of actual geometry and their respective optimal values (that are fully determined by a number of fixed geometric values which are not adaptable, such as the grating dimensions, the spot size of the X-ray source, and the dimensions of the digital image detector).

The calculation disclosed with this invention allows for subsequent adjustment of the variable parameters in order for the PCI system to produce optimal images, as set out in claims.

The above aspects are solved by a method as set out in claims 1 - 11.

In the context of this invention, a PCI system is a coded aperture phase contrast imaging system that comprises the following essential components: an X-ray source, a pre-sample mask (or coded aperture) and a digital image detector fitted with a detector grating. The detector grating may be replaced by a so-called virtual grating, wherein the physical detector grating is substituted by a selective column read-out technique of the digital image detector which mimics the behaviour of a physical grating. The application of such virtual grating allows for the use of an X-ray detector with a larger spot size which then allows the use of conventional X-ray sources.

X-rays emitted from the X-ray source are shaped into individual beamlets by means of selective absorption by the pre-sample mask or "coded aperture" (which is an absorbing grating). The pre-sample mask allows only narrow portions of the X-ray beam to pass through so-called apertures of identical width. The pre-sample mask typically has a fixed geometry that is adapted to a detector grating or analyser grating that in its turn is fitted to the geometry of the used X-ray detector pixel grid dimensions while taking into account the distances between the X-ray source, pre-sample mask and X-ray detector. The pre-sample mask has a pitch size $p$. The X-ray detector or digital image detector is characterized by a pixel pitch size $s_p$, so that the detector grating has a pitch size $s_p.n$ wherein $n$ is an integer. The fixed geometry of the pre-sample mask and the detector pixel grid therefore determine to a large extent the theoretical geometrical setup of the entire system (i.e. the relative positions of the components in the system).

As mentioned above, the method provides for the calculation of the deviation from the correct setting for 3 adjustable parameters of the geometry of such a PCI system, after that the fixed design parameters of the system (geometry of the pre-sample mask, the detector and the detector grating) have been determined. These 3 adjustable parameters are:

> 1) the linear position of the pre-sample mask along the linear axis between the X-ray source and the centre of the digital image detector (i.e. the adjustment of the magnification of the system),
> 2) the phase alignment of the projected shadow of the pre-sample mask with the detector mask of the digital image detector (i.e. the adjustment of the starting point of the phase contrast image), and
> 3) the angular alignment of the pre-sample mask with detector mask (i.e. the adjustment of the angle between the pre-sample mask grating and the detector grating),

The method relies on a so-called adjustment image, which is an image that is the result of an acquisition performed on the uncorrected or non-adjusted PCI system by making an exposure without there being an object in position to be imaged. In other words the adjustment image is what is called a flat field exposure in conventional X-ray imaging but with the pre-sample mask in the beam path. The same adjustment can be used to calculate the deviations for all 3 adjustable parameters from their respective theoretical optimal value.

An intensity profile is a 1-dimensional measurement along a single line of pixels of the digital image detector.

Some of the adjustment measurements will require that the measured pixel line is aligned almost in parallel or either perpendicular to the apertures of the pre-sample mask (and detector mask).

Specific examples and preferred embodiments are set out in the dependent claims.

The present invention is beneficial in that the method allows to calculate the required adjustments of the 3 adjustable parameters based on the data of a single or at most two adjustment image acquisitions. While the measurement method allows the calculation of the absolute value required for the correction, it does not provide the direction into which the adjustment needs to be made. This means that in case that a first calculation would have been followed by an adjustment of any of the 3 adjustable parameters in the wrong direction, measurement of a second adjustment image would reveal that the adjustment of said parameters would have worsened the off-set or (in a best case) corrected the deviations.

In the context of this invention, the term "substantially" used in combination with geometric comparative features such as "parallel", "perpendicular", etc... should be understood that the geometric relation between two objects should be aligned as such ("parallel", "perpendicular", etc...) to each other within a certain tolerance that still allows our method to perform successfully. In this context, accepted tolerances of angles should be understood to be in the order of magnitude of 1° or lower, but not exceeding 5°.

Further advantages and embodiments of the present invention will become apparent from the following description and drawings.

## Brief description of drawings

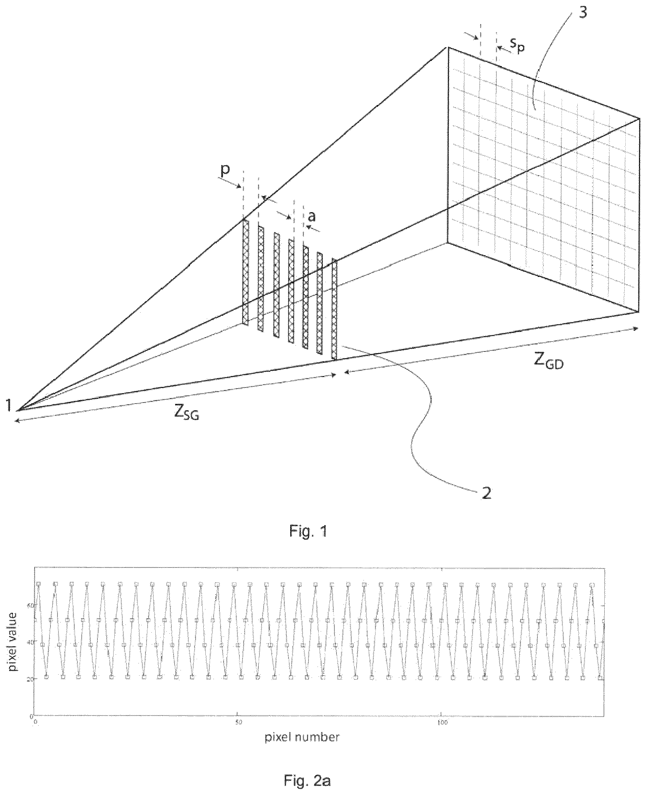

Fig. 1 gives a schematic overview of a coded aperture phase contrast imaging system, comprising an X-ray source [1], a pre-sample mask [2] and digital image detector [3]. The pre-sample mask has a pitch size $p$ and aperture size a. The pre-sample mask [2] is positioned orthogonally to the X-ray beam produced by the X-ray source [1], and parallel to the digital image detector [3]. The digital image detector has a pixel pitch size $s_p$. The pre-sample mask [2] is positioned at a distance $Z_{SG}$ from the X-ray source, and at a distance $Z_{GD}$ from the digital image detector [3].

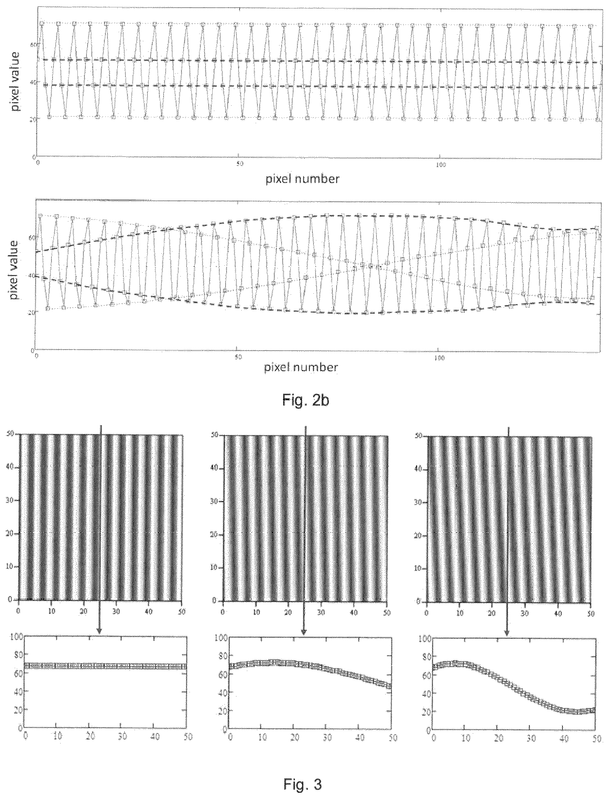

Fig. 2 represents a simulated intensity profile of a single line of the detector. The squares represent measurement points of the intensity profile collected by each image detector pixel. In this simulated example, the magnification and grating pitch are selected to produce a projected grating pattern with a pitch of 4 pixels; a full period of the pre-sample mask grating is captured by 4 detector pixels in this example.

Fig. 3 illustrates the effect of angular misalignment of a projected image produced by a pre-sample mask on a digital image detector (upper images), and the effect on an intensity profile that is retrieved in parallel with the grating bars (or virtual grating lines) of the detector mask.

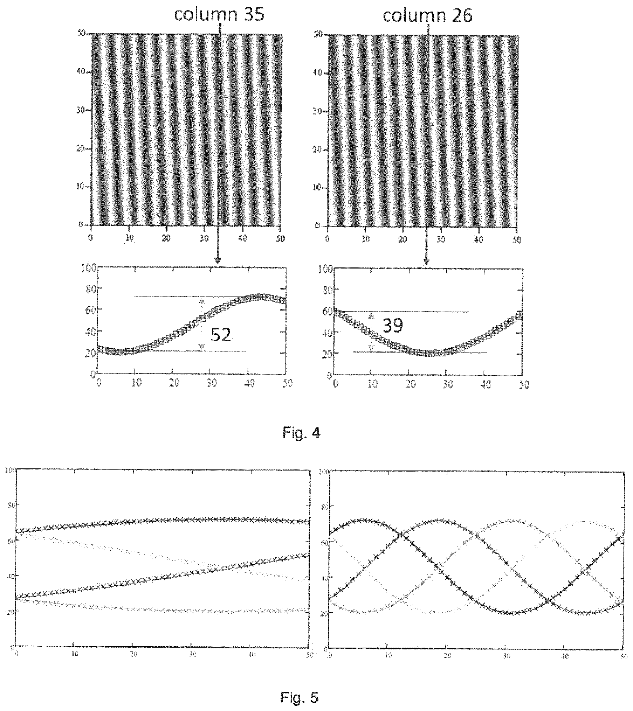

Fig. 4 illustrates how the maximum variation along the intensity profile (collected in parallel to the grating bars) is to be determined in order to subsequently measure the period of this variation. This period is a measure of the angular deviation between the pre-sample mask and the detector mask.

Fig. 5 is a graph of 4 neighbouring intensity profiles extracted from 4 neighbouring pixel lines of a detector in the direction of the virtual grating bars, given that the period of the virtual grating is 4 in this example. The intensity profiles repeat themselves with a period of 4 pixels. The left graph illustrates a case where there is a small angular variation to be measured (large period), whereas the right graph illustrates a case where there is a larger angular variation (small period). As can be seen, the period of the left graph is a lot larger than the period in the right graph, which illustrates that the period of this graph is inversely proportional to the angular deviation; large period confers to small angular deviation.

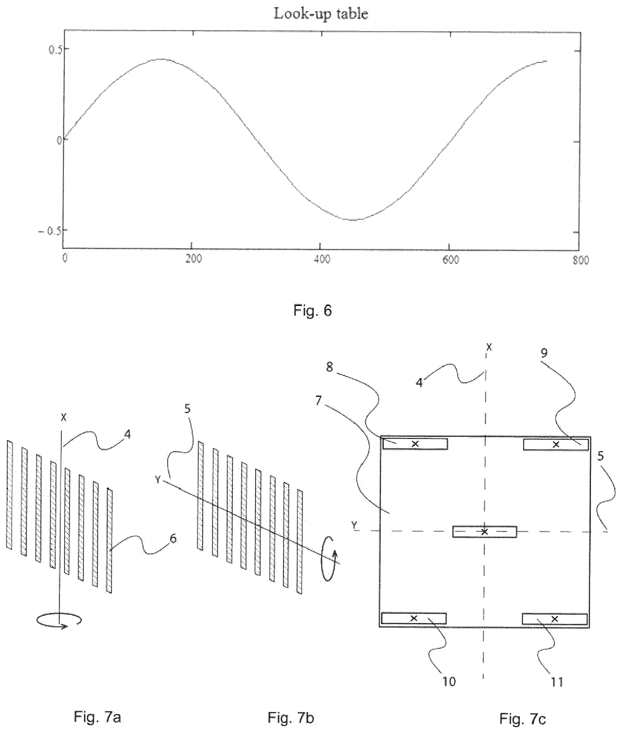

Fig. 6 gives an example of a look-up table generated in advance that stores the look-up table values ($LT$) correlated with measured lateral positional errors.

Fig. 7a and 7b respectively illustrate the tilt directions around the 2 possible orthogonal axes in the plane of the pre-sample mask. Fig. 7a represents the tilt direction around the orthogonal axis (4) in parallel with the grating bars (6) of the pre-sample mask, whereas Fig. 7b represents the tilt direction around the orthogonal axis (5) perpendicular to the direction of the grating bars (6) of the pre-sample mask.

Fig. 7c illustrates the detector (7) indicating a number of preferred areas (8,9,10,11) on the detector of which the magnification errors can be determined to further calculate the tilt angle of the detector. For this calculation, also the distances between the centres of said areas (indicated by the "x") have to be determined.

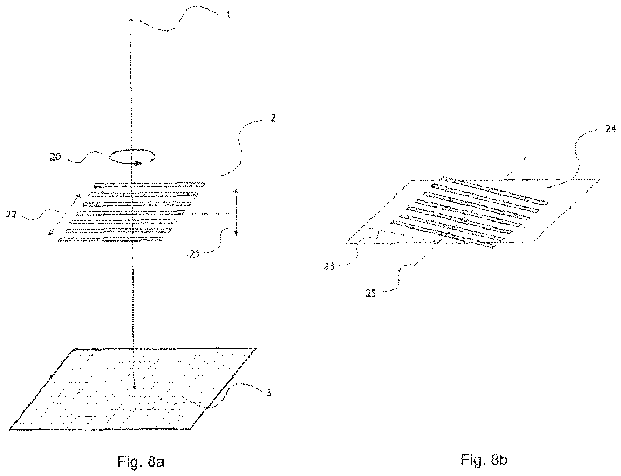

Fig. 8a illustrates the relative positioning of the main components of a PCI system, and the different types of alignment error that may be associated with the position of the pre-sample mask (2). The main components are indicated: X-ray source (1), digital image detector (3), and the pre-sample mask (2) that is oriented perpendicularly to the axis between the X-ray source and the centre of the digital image detector. Further indicated in the drawing are the 4 different parameters that may be adjusted with an impact on the relative position of the pre-sample mask in the PCI system configuration:

(i) the angular difference (20) between the pre-sample mask and the detector grating (i.e. the angle between the pre-sample mask grating and the detector grating in-plane of both masks),
(ii) the linear position (21) of the pre-sample mask along the linear axis between the X-ray source and the centre of the digital image detector (i.e. the adjustment of the magnification of the system),
(iii) the phase alignment (22) of the projected shadow of the pre-sample mask with the detector mask of the digital image detector (i.e. the adjustment of the starting point of the phase contrast image), and
(iv) the tilt angle (23) of the pre-sample mask with respect to a plane (24) that is perpendicular to the line between the X-ray source and the centre of the image detector. The tilt angle (23) in the drawing is the tilt angle about the Y-axis (25), which is oriented perpendicular to the grating bars of the pre-sample mask (Fig. 8b). But also the tilt angle about the X-axis can be measured and corrected .

## Description of embodiments

In the following detailed description, reference is made in sufficient detail to the above referenced drawings, allowing those skilled in the art to practice the embodiments explained below.

In order for conventional X-ray systems to be adapted (or retrofitted) to be able to be used as a PCI system, a number of design choices should be made in order to optimize the resulting image quality obtainable by the system. These design choices concern adaptations of the design and/or the positioning of certain components to the limitations and geometries of components of the conventional X-ray system which are going to be reused in the PCI system. As will follow from the explanations below, a typical PCI system requires at least very precisely manufactured so-called masks or gratings. These masks or gratings are precision components as they need to be adapted to either directly fit to the pixel structure of the digital image detector matrix or will have to cast their shadow to fit to this pixel structure.

A first design choice is to use a pre-sampling mask with a pitch $p$ adapted to the focal spot size (p >250 $\mu$m) of the X-ray tube of the conventional X-ray modality. The influence of the pre-sampling mask pitch on the system sensitivity for a given focal spot size of the x-ray tube is described in "Peter R.T. Munro, Konstantin Ignatyev, Robert D. Speller and Alessandro Olivo - 1 March 2010 / Vol. 18, No. 5 / OPTICS EXPRESS 4103". As a conclusion, the pitch of pre-sampling mask should be chosen larger than 200 $\mu$m given that the focal spot size of standard x-ray tubes is typically larger than 250 $\mu$m.

Secondly, a so-called magnification factor is chosen for the system so that the projected pitch of the pre-sampling mask is equal to an integer multiple of the pixel size (the magnification of the system must be adjusted accordingly). In other words, the magnification factor has an impact on the magnification of the imaged object on the digital detector image, but also has a consequence on the relative distances between the X-ray source and pre-sample mask ($Z_{SG}$), and the pre-sample mask and digital image detector ($Z_{GD}$) respectively. The condition that the projected pitch $p' = M.p$ on the x-ray detector is equal to an integer multiple of the pixel size must be fulfilled very strictly. This leads to a second design condition for the PCI system: magnification $M$ of the pre-sampling mask must be an integer $n$ multiple of the ratio between pixel size $s_p$ and grating pitch: $M = n \cdot s_p/p$. Due to the fact that the parameter $s_p/p$ is fixed and defined by the design of the pre-sampling mask and the detector, the magnification $M$ must be fine-tuned each time the system is reconfigured to PCI to fit the condition for $n$.

Finally, in order to overcome the physical design difficulties with the detector grating, it is preferred to implement a virtual detector grating, avoiding the hurdles associated with the adjustment and manufacturing of such detector gratings. Detector gratings or analyser gratings are quite fragile components as they are required to cover the entire digital imaging detector surface and thus have a considerable size while at the same time having a very fine structure. These grating must be aligned very accurately to the pixels of the detector i.e. parallel to the pixel columns with a phase of $\pi/2$.

A virtual detector grating solves this problem as it differs from a physical grating in that a virtual detector grating constitutes of a standard image detector but that is used in a different way. Instead of using a physical detector grid blocking part of the detector pixels, the detector pixels are grouped in a special manner and only a part of the total pixels are used to calculate the absorption, phase image and dark field image. The recalculation is done by sequentially using the pixels which were not used in the previous sub-image. In this case the required 3 intensities $I_{1...3}$ can be deduced from a single exposure. In a second, third... step the pre-sample mask can be moved to calculate additional phase contrast images as well as absorption and dark field images to increase resolution if required. Finally the different sub-images can be fused to result in full resolution images for phase, absorption and dark field. The advantage of this method is that no physical mask or grating is required suffering from certain design and manufacturing constraints.

Conclusively, considering the design choices mentioned before and thanks to the proposed calibration methods presented further in this disclosure, it comes within reach to adapt a conventional X-ray device such that the main components (such as the X-ray generator, X-ray tube, digital image detector and the components driving them) can be reused as main components for a PCI setup. Important adaptations will nevertheless be required in order to ensure sufficient mechanical stability and sufficient positional accuracy for the desired geometry to be achieved. For instance it is required that the 3 main components of the PCI system (X-ray source, pre-sample mask and digital image detector (comprising the detector grid)) may be positioned and fixed in space in a very specific geometric relationship with each other, and this within sub-millimetre accuracy.

In order to ensure sufficient mechanical stability of the pre-sampling mask and the digital image detector in the existing X-ray configuration, the PCI configuration is preferably complemented with three additional parts: 1) a support for the pre-sampling mask comprising an alignment system to align the mask, 2) a detector support and 3) a tube interface to fix the collimator tube assembly to the PCI setup. Preferably the PCI assembly has mechanical adjustment interfaces that allow coarse pre-adjustment within mm accuracy of the positions of the pre-sample mask the digital image detector and the tube respectively. The final and very accurate adjustment is then performed after this coarse adjustment, making use of the method of the invention as disclosed in detail below.

So, before any adjustments are made, first the X-ray source is positioned opposite to the digital image detector and is positioned at a pre-defined but not exactly known distance $Z_{SG} + Z_{GD}$ from the source. The image detector is oriented almost perpendicularly to the main beam of the X-ray source, and determines a linear axis between the X-ray source and the centre of the digital image detector. As explained above, design choices have been made with regards to the desired magnification of the system, which defines the requirements for the position of the pre-sample mask in the system, as well as its design (i.e. defined by its pitch size $p$ and aperture size $a$). The pre-sample masks' projected pitch size on the digital image detector should be matched to the actual pitch size (or a multiple the actual pitch size) of the digital image detector.

The position of the pre-sample mask in the PCI system is therefore entirely determined by the choice of the magnification and by the distance between the X-ray source and the digital image detector. The characteristics of the digital image detector specify the dimensions (pitch size $p$ and aperture size $a$) of the pre-sample mask. The theoretical position of the pre-sample mask in the PCI geometry is therefore known, which is at a distance $Z_{SG}$ from the X-ray source and at a distance of $Z_{GD}$ from the digital image detector. The pre-sample mask should be aligned to the orientation of the pixel matrix of the digital image detector in order to achieve the intended image quality. This means that the pre-sample mask should be positioned in parallel to the digital image detector (and thus its surface should be oriented perpendicularly towards the X-ray main beam). Also, the grid structure (parallel grating slits separated by grating bars) should be aligned with the orientation of the detector grid (or the pixel grid of the digital image detector).

The alignment method for correcting the total geometrical setup of the PCI system thus comprises adjustments to the available degrees of freedom of the pre-sample mask in space in relation to the X-ray source and digital image detector. The adjustments to the degrees of freedom can be expressed as adjustments to 4 possible geometric parameters that are fully determined by the relative positions of the pre-sample mask and the digital image detector in combination with the X-ray source. These 4 parameters are the following:

(i) the angular alignment of the pre-sample mask with the detector grating about the orthogonal axis of said masks (i.e. the adjustment of the angle between the pre-sample mask grating and the detector grating in-plane of both masks),
(ii) the linear position of the pre-sample mask along the linear axis between the X-ray source and the centre of the digital image detector (i.e. the adjustment of the magnification of the system),
(iii) the phase alignment of the projected shadow of the pre-sample mask with the detector mask of the digital image detector (i.e. the adjustment of the starting point of the phase contrast image), and
(iv) the tilt angle (23) of the pre-sample mask with respect to a plane that is perpendicular to the line between the X-ray source and the centre of the image detector.

In order to support and facilitate the physical magnification, position and angular adjustments which follow the calculations, the pre-sample mask or grating assembly and the digital image detector support shall contain means to move the respective masks (or gratings) along an axis perpendicular and parallel to the grating plane by a pre-defined distance as well as a rotation mechanism. This mechanism should allow both coarse and very fine positional adjustments, which means that special care should be taken when designing the supporting components and holders.

The adjustment X-ray image is taken with the pre-sample mask and detector support in place. It is herewith assumed that the coarse adjustments (to within millimetre accuracy) have been made prior to the execution of the herewith disclosed accurate correction method, in order for the method to reveal the required corrections based on a single adjustment X-ray image. If this coarse adjustment is or cannot be executed accurately enough, further iterations may be required to achieve the optimal geometric setup, requiring more acquisitions of adjustment X-ray images.

In the method disclosed here, the adjustments of the distances and the angles are calculated advantageously from a single adjustment X-ray image. The method thus allows under certain conditions that the corrections are calculated one after the other without the need for additional exposures. This is however subject to some limitations wherein the required adjustment would exceed certain values.

As a first common step in the different processes to adjust any of the 4 parameters mentioned above, the so-called adjustment X-ray image is made. The adjustment X-ray image will be the basis for any of the subsequent calculations. During the acquisition of the adjustment X-ray images, the digital image detector should be set to full resolution so that each exposed detector pixel generates an image pixel. No object shall be between pre-sample mask and image detector - apart from air and object support. As such, the pre-sample mask produces an image (2 dimensional) of its projected grating structure. Periodic intensity profiles (1 dimensional) may be extracted from the adjustment image in the two directions of the digital image detector, by extracting all pixel values of a line of pixels of the detector. The intensity profiles will be used to measure certain characteristics that relate to different aspects of the position and orientation of the pre-sample mask.

Before extracting the intensity profiles from the adjustment image to apply them in the correction method disclosed here, it may be advantageous to first normalize the image. Normalization of the image changes the range of pixel intensity values, with the purpose to optimize the used value range in the profile. Additionally, a shading correction may be considered in order to compensate inhomogeneity's of the X-ray beam.

The intensity profile on which the subsequent calculations are based is then obtained by extracting the pixel values from a single line from the adjustment image in the required direction (the direction X or Y varies depending on which parameter deviation is being calculated). In principle, any of the pixel lines in the adjustment image is suitable as an intensity profile as their results should be identical. A sample of such an intensity profile is depicted in Fig. 2. After acquiring the required intensity profile from the adjustment image, the different calculations of the adjustment parameters followed by corresponding corrections may be applied to the system.

After being calculated, these corrections may then be applied to the PCI system in two different ways; either by physically correcting the system setup by physically moving at least one of the components and thus compensating for the measured deviation, or else, by digitally compensating the error in the subsequently acquired images of the subject (or patient) prior to applying any reconstruction algorithms or with an integrated algorithm. Using standard methods the latter is however only possible for compensation of positioning errors of the pre-sample mask that are oriented "in-plane" of the pre-sample mask (i.e. for correction of the alignment of the pre-sample mask with the detector grating, and the phase alignment of the projected shadow of the pre-sample mask with the detector mask of the digital image detector). Standard digital corrections to acquired images cannot compensate errors with an impact on the magnification of the PCI system. Standard digital correction of images thus can compensate for instance rotation and displacement errors in the plane of the pre-sample mask in the same way a physical correction would correct these deviations. To digitally compensate all adjustment errors more dedicated compensation algorithms are required which are adapted to the physical characteristics of the PCI assembly.

In the next section, the 4 different methods (i) to (iv) for calculating the different deviation parameters are explained in more detail. All these methods are based on information or a characteristic of one or more intensity profiles.

(i) First, in a preferred embodiment, an error of the angular alignment of the pre-sample mask with the detector grating around an orthogonal axis of said masks (i.e. the adjustment of the angle between the pre-sample mask grating and the detector grating in-plane of both masks) may be calculated based on the analysis of an intensity profile that is extracted from the adjustment image in the direction of the grating bars (i.e. the extracted line of pixel values parallel to the grating bars). To calculate the rotation angle of the offset, the variation along a column has to be used in order to get the frequency $f$ of the periodic variation of the pixel values (preferably a column with maximum modulation shall be selected).

$$pv_i = a.\sin(f.i) + o$$

Next, the period length of this sequence from the frequency period = 1/f is calculated e.g. by a least square fitting method. Dividing the length of the detector by the period length gives the number of periods fitting into the detector: N, which is not an integer value. The deviation angle is then given by

$$\alpha = arctan\left[\frac{(N.s_p)}{L}\right]$$

, wherein $L$ is the detector length, and $s_p$ is the projected pitch of the pre-sample mask. This process should ideally be repeated for different intensity profiles extracted from different pixel columns of the detector.

In addition to the above method to calculate the angular deviation, also the direction of the deviation (clockwise or counter clockwise) may be extracted. In this approach the position of a single beamlet is calculated for different rows of the detector as disclosed in (iii) "lateral adjustment" further down in the text. In case a tilt error is present the beamlet centre position changes almost linearly with increasing row number (perpendicular to grating bars). The slope $sl$ of this change can be used to estimate the tilt. In case of a larger tilt error a saw tooth like curve will be found as the intensity profile is periodic. The slope of a single tooth $sl$ can be used to calculate the angle in this case. $\alpha = arctan(sl)$. To increase accuracy the slope of all "teeth" can be averaged. The sign of the tilt angle $\alpha$ determines the rotation direction.

The calculated angle $a$ is subsequently used for correcting the incorrectly aligned PCI system by physically adjusting the angle between the pre-sample mask and the digital image detector mask. In the case that this correction is applied, a new adjustment image has to be taken in order to apply further correction steps of the described (complete) procedure. However, it is also possible to avoid the need for this additional acquisition: it is possible to apply the calculated angle $a$ as a digital correction to the original adjustment image, and using this digitally corrected (rotated) image in any of the further adjustment steps (ii) to (iv).

(ii) Second, in another preferred embodiment, another adjustment step involves the correction of the magnification of the PCI system, that can be calculated based on the analysis of an intensity profile that is extracted from the adjustment image in the direction perpendicular to the direction of the grating bars (i.e. the extracted line of pixel values is perpendicular to the grating bars). The magnification is determined by the relative position of the pre-sample mask on the axis between the positions of the X-ray source and the digital image detector, and by the position of the object to be imaged along this axis. In the context of this invention, only the relative position along the axis between the positions of the X-ray source and the digital image detector are considered. As the magnification stands in relation to the pitch of the pre-sample mask and the pitch of the digital image detector (the projected pitch of the pre-sample mask must coincide with the pitch of the detector), the intended position of the pre-sample mask is pre-determined by design choices on the system. The magnification error therefore has to be interpreted as the deviation between the actual and the intended position of the pre-sample mask along the axis between the X-ray source and the centre of the digital image detector. The magnification M is given by

$$M = \frac{Z_{SG} + Z_{GD}}{Z_{SG}}$$

The magnification must be set with an accuracy better than 0.002% (better than $2.10^{-5}$). The distances $Z$ are typically in the range of 1 meter thus the required accuracy of positioning in z-direction should be 20 $\mu$m or better.

A wrong setup of the magnification of the PCI system results in a beating (Moiré) between the spatial frequency of the projected grating pattern and the pixel pattern. This effect is visible in the measured intensity profile and the frequency $f_B$ of this beating pattern is measurable. Fig. 2b illustrates such a beating pattern in the intensity profile. The beating frequency $f_B$ is a measure for the deviation from the desired value of the magnification of the PCI system. A correctly aligned PCI system, having the correct magnification for which it was designed, results in no beating in the intensity profile (as illustrated in Fig. 2a).

The pitch $p'$ of the projected grating pattern on the panel is $p \cdot M$ ($p$ is the pitch of the grating and $M$ the magnification). The pitch of the virtual grating is $s_p \cdot n$ ($s_p$ is the pixel size and $n$ is an integer). The spatial frequencies and the beating frequency $f_B$ are given as:

$$f_G = \frac{2\pi}{p \cdot M} \quad and \quad f_P = \frac{2\pi}{s_p \cdot n} \;; \quad f_B = |f_G - f_P|$$

$$M' = \frac{2\pi \cdot s_P \cdot n}{2\pi \cdot p \pm f_B \cdot p \cdot s_p \cdot n}$$

on the other hand:

$$M = \frac{Z_{SG} + Z_{GD}}{Z_{SG}}$$

$Z_{GD} + Z_{SG}$ is not changed when moving the grating along the main X-ray beam as $\Delta Z_{GD} = -\Delta Z_{SG}$. From the real magnification M' resulting from the real geometry $Z_x^{real}$ the required source to grating distance $Z_{SG}^{req}$ can be estimated: $\frac{M}{M'} = \frac{Z_{SG}^{real}}{Z_{SG}^{req}}$; the real source grating distance can be approximated by its design value.

(iii) Third, in another preferred embodiment, the positioning accuracy for lateral adjustment depends on the pitch of the pre-sample grating and should be better than p/40 - for typical pitch values this is in the range of 10 μm. To estimate the relative lateral position error, first, an intensity profile is extracted from the adjustment image in the direction perpendicular to the direction of the grating bars (i.e. the extracted line of pixel values is perpendicular to the grating bars). The rolling binning value I of the at least first 8 pixels in this intensity profile from the single pixel value pv is calculated by:

$$I_k = pv_k + pv_{k+1}$$

k is the number of a pixel.
Next, we calculate:

$$C = -2\ln\left(\frac{I_1}{I_2}\right); D = -2\ln\left(\frac{I_3}{I_2}\right);$$

The lateral deviation μ is then calculated as:

$$\mu = \frac{s_p}{2}\left(\frac{D-C}{D+C}\right)$$

$s_p$ is the pixel size.

In yet another embodiment, another approach is taken to estimate the lateral positional accuracy of the pre-sample mask based on a comparison with a look-up table that is produced for a particular system during manufacturing or installation. With this approach, there is an advantage that the method uses the real intensity profiles of the system, rather than the mathematical approximations used in the mathematical approach explained here above. The method is based on the characterisation of a PCI system during production or installation, by calculating a look-up table that can be used for later comparison against it. The look-up table values are calculated upfront for each reference image that is measured on the system, and that corresponds to a known lateral positional displacement error. The characterisation is done as follows.

First, a set of images is generated upfront during production or at installation of the system wherein the pre-sample mask is offset with a known lateral displacement error. From this set of images, a look-up-table value is calculated for each simulated lateral positional error. This is done as follows:

Calculate the rolling binning value I of the at least first N pixels from the single pixel value pv by:

$$I_k = pv_k + pv_{k+1},$$

k is the number of a pixel. Next, for each image with its corresponding lateral positional displacement error, a look-up table value LT is calculated:

$$LT = \frac{I_n - I_{n+2}}{I_{n+1} + I_{n+3}}$$

wherein n is an integer which defines the reference pixel which corresponds to a position difference of 0. After this, the look-up table is interpolated and stored for later comparison.

When the look-up table is available after the characterization steps above, any lateral positional error may be easily obtained by extracting an intensity profile from an adjustment image in the direction perpendicular to the direction of the grating bars (i.e. the extracted line of pixel values is perpendicular to the grating bars). Subsequently, the reference value *ref* is calculated as follows:

$$ref = \frac{I_n - I_{n+2}}{I_{n+1} + I_{n+3}}$$

wherein the rolling binning value *I* of the at least first *N* pixels from the single pixel value *pv* is calculated by:

$$I_k = pv_k + pv_{k+1}$$

*k* is the number of a pixel. This value *ref* is then compared against the look-up table in order to find the corresponding lateral positional error.

(iv) Fourth, and in yet another preferred embodiment, another adjustment step concerns the correction for a tilt angle of the pre-sample mask with respect to a plane that is perpendicular to the line between the X-ray source and the centre of the image detector. The tilt angle can be decomposed into two tilt angles $\beta_x$ and $\beta_y$ respectively tilting around 2 orthogonally oriented X- or Y-axes in the plane and parallel to the edges of the pre-sample mask. The tilt angle is the deviation from a fully perpendicular orientation of the pre-sample mask to the line between the X-ray source and the centre of the image detector. The tilt angle should in principle be 0 (zero) in order to have a correctly aligned PCI system. The X-axis is oriented in parallel with the grating bars, whereas the Y-axis is oriented perpendicular to the grating bars of the pre-sample mask.

These tilt angles can be calculated based on the analysis of a series of (at least 2) intensity profiles that are extracted in selected areas in the adjustment image as illustrated in Fig. 7c as (8, 9, 10, 11). The intensity profiles are extracted in the direction perpendicular to the direction of the grating bars (i.e. the extracted line of pixel values making up the intensity profile is perpendicular to the grating bars).
The method is based on the calculation of the difference between the magnification errors in at least 2 different areas of the detector, which are preferably located at opposite edges of the detector and mirrored around X- or Y-axis for which the tilt (respectively $\beta_x$ and $\beta_y$). In order to calculate the tilt angle $\beta_x$, the magnification error $M_1$ in the area (8) in combination with the magnification error $M_2$ in area (9) should be calculated. Alternatively, the magnification error $M_1$ in the area (10) in combination with the magnification error $M_2$ in area (11) could be calculated. In order to calculate the tilt angle $\beta_y$, the magnification error $M_1$ in the area (8) in combination with the magnification error $M_2$ in area (10) should be calculated. Alternatively, the magnification error $M_1$ in the area (9) in combination with the magnification error $M_2$ in area (11) could be calculated. From the difference between the measured magnification errors (determined by the method as described under (ii)) and the distance between the centres of the 2 measured areas, the tilt angle $\beta$ is calculated as:

$$\beta = \arctan\left(\frac{(M_1 - M_2) \cdot Z_{SG}}{l}\right)$$

wherein $M_1$ is the magnification error in a first area of the detector, $M_2$ is the magnification error in a second area of the detector, $Z_{SG}$ is the design value for the source grating distance and *l* the distance between the centres of the 2 measured areas on the detector.

Preferably, and in order to achieve the best results, a preferred order for tackling the different adjustments is recommended. This approach is however only relevant in case the alignment errors of the (uncorrected) starting position of the pre-sample mask exceeds certain thresholds. In case these errors remain under these threshold values, a single exposure may suffice to perform all 4 adjustment calculations.

In a preferred embodiment, it is therefore advantageous to first calculate the angular alignment error between the orientation of the pre-sample mask and the orientation of the detector mask according to the above disclosed method. In case the angular error exceeds 0.25°, there is an impact on the accuracy of the calculation of the magnification error. This is caused by a change of the source to grating distance at different positions of the detector (for a typical geometry 0.25° lead to a change of ~100 $\mu$m which is 5 times the required accuracy). The real magnification M' thus is a function of the position on the detector. This leads to a varying beating frequency along the detector pixel line. The optimum method is to use only pixels which are close to the rotation axis to calculate M' but the number of pixels should still be sufficiently large to get a good estimate of the beating frequency; which is at least 20 % of the total pixels in a row. In case the error lies between 0.5° and 1°, it is recommended to digitally correct (i.e. digitally rotate) the adjustment image by the obtained result from the calculation before using this digitally corrected (rotated) adjustment image again in a subsequent step. In case that the error is larger than 1°, a physical correction of the rotation angle is preferred. I.e. in this case it is preferred to physically realign the pre-sample mask prior to acquire a second adjustment image that may be used in subsequent calculations.

In a preferred embodiment, it is advantageous to take a similar approach on the calculation of the deviation of the second parameter; the magnification. The upper threshold that is allowable for direct correction by the disclosed method is 0.1% of the desired magnification, which means that in case a larger than 0.1% magnification is measured, it is for purposes of achieving the best end result advantageous to first improve the physical alignment of the pre-sample mask prior to acquiring a new adjustment image. The newly acquired adjustment image should then have an acceptable error for which the error of the magnification does not exceed the threshold (and thus may be corrected by the disclosed error correction method).

**Claims**

1.  Method for alignment optimization of a pre-sample mask in a PCI system, said PCI system comprising an X-ray source, a pre-sample mask and a digital image detector comprising a detector grating, the method comprising the steps of:

    - acquiring an adjustment image by making an exposure with said PCI system,
    - extracting at least one intensity profile from said adjustment image that corresponds to a signal profile of a line of pixels on said digital image detector,
    - calculating and correcting at least one of the following positional deviations (i) to (iv) of said pre-sample mask based on a characteristic of said intensity profile:

        (i) an angular alignment error determined as a deviation of an angle between the orientation of said pre-sample mask and the orientation of said detector grating, wherein said characteristic is a period length or a multitude of relative differences between 2 consecutive binned pixel pairs of said intensity profile;
        (ii) a magnification error determined as a deviation of a position from an intended position of said pre-sample mask and its actual position along a radiation axis, wherein said characteristic is a beating frequency of said intensity profile;
        (iii) a lateral displacement error determined as a deviation between a projected grating pattern of said pre-sample mask and a corresponding grating pattern of said detector grating, wherein said characteristic is the relative difference between 2 consecutive binned pixel pairs of said intensity profile; or
        (iv) a tilt error determined as an angular deviation about 2 orthogonal axes in plane of said pre-sample mask from a plane perpendicular to a radiation axis, wherein said characteristic is a combination of beating frequencies in multiple intensity profiles selected from different areas of said digital image detector.

2.  Method to optimize the alignment of a pre-sample mask in a PCI system according to Claim 1, said PCI system comprising an X-ray source positioned at a distance $Z_{SG}$ from said pre-sample mask, said PCI system comprising a digital image detector with length L positioned at a distance $Z_{GD}$ from said pre-sample mask, said digital image detector **characterized by** a pixel pitch size $s_p$ and a detector grating pitch size $s_p.n$ wherein $n$ is an integer value, said pre-sample mask having a pitch size p, **characterized in that** said angular alignment error is calculated and corrected by the steps of:

    - extracting an intensity profile from said adjustment image that corresponds to a signal profile of a single line of pixels on said digital image detector, said single line of pixels being oriented substantially parallel to the direction of the orientation of the apertures of said pre-sample mask of said PCI system,
    - determining a period $p_1$ of a periodic variation in said extracted intensity profile,
    - calculating a rotational error about a perpendicular axis of said pre-sample mask as an angle between said pre-sample mask and said digital image detector,
    wherein said error is given by;

$$a = \arctan\left[\frac{(N. \text{projected pitch})}{L}\right]$$

wherein N is the number of periods fitting in detector length L

$$N = \frac{L}{p_I}$$

- applying the angle $a$ to said pre-sample mask to perform said rotational adjustment.

3. Method to optimize the alignment of a pre-sample mask in a PCI system according to Claim 1, said PCI system comprising a digital image detector positioned at a distance $Z_{GD}$ from said pre-sample mask, said detector of which a detector grating is oriented in parallel with the apertures of said pre-sample mask, said detector being **characterized by** a pixel pitch size $s_p$ and said detector grating being **characterized by** a pitch size $s_p.n$ wherein $n$ is an integer value, the pre-sample mask having a pitch size p, said PCI system comprising an X-ray source positioned at a distance $Z_{SG}$ from said pre-sample mask, **characterized in that** said angular alignment error is calculated and corrected by the steps of:

- extracting multiple intensity profiles $m$ from said adjustment image that correspond to signal profiles of single lines of pixels of said digital image detector, said single lines being oriented substantially perpendicular to the direction of the orientation of the apertures of said pre-sample mask of said PCI system,
- calculating a rolling binning value $I$ of at least first 8 pixels from single pixel values $pv_k$ as $I_k = pv_k + pv_{k+1}$ of each line; $k$ being the order number of a pixel in said intensity profile;
- calculating a lateral deviation $\mu(m)$ from an optimal lateral position for each line $m$ as;

$$\mu = \frac{s_p}{2} \cdot \frac{D - C}{D + C} \quad ,$$

wherein

$$C = -2\ln\left(\frac{I_1}{I_2}\right); D = -2\ln\left(\frac{I_3}{I_2}\right);$$

and $s_p$ is a pixel size of the digital image detector,

- calculating an angle $a$ from the slope $sl$ of $\mu(m)$ by $\alpha = \arctan(sl)$
- applying the angle $\alpha$ to said pre-sample mask to perform said rotational adjustment.

4. Method to optimize the alignment of a pre-sample mask in a PCI system according to Claim 1, said PCI system comprising an X-ray source positioned at a distance $Z_{SG}$ from a pre-sample mask, said PCI system comprising a digital image detector positioned at a distance $Z_{GD}$ from said pre-sample mask, and **characterized by** a pixel pitch size $s_p$ and a detector grating pitch size $s_p.n$ wherein $n$ is an integer value, said pre-sample mask having a pitch size p, said intended magnification $M$ of said PCI system being determined by;

$$M = \frac{S_p.n}{p}$$

and the geometry of said PCI system being determined by

$$M = \frac{Z_{SG} + Z_{GD}}{Z_{SG}}$$

**characterized in that** said magnification error is calculated and corrected by the steps of:

- extracting an intensity profile from said adjustment image that corresponds to a signal profile of at least a part of a single line of pixels of said detector, said single line being oriented substantially perpendicular to the direction of the orientation of the apertures of said pre-sample mask of said PCI system,

- retrieving a beating frequency $f_B$ from said intensity profile by measuring the frequency of the interference (Moire) pattern,
- calculating a deviation from the optimal magnification M by calculating the actual magnification $M'$;

$$M' = \frac{2\pi . S_p . n}{2\pi . p \pm f_B . p . S_p . n}$$

- applying a position shift $\Delta Z_{SG} = Z_{SG} . \left(1 - \frac{M}{M'}\right)$ to said pre-sample mask to perform said magnification adjustment.

5. Method to optimize the alignment of a pre-sample mask in a PCI system according to Claim 1, said PCI system comprising a digital image detector positioned at a distance $Z_{GD}$ from said pre-sample mask of which a detector grating is oriented substantially parallel with the apertures of said pre-sample mask, said digital image detector being **characterized by** a pixel pitch size $s_p$ and said detector grating being **characterized by** a pitch size $s_p . n$ wherein $n$ is an integer value, the pre-sample mask having a pitch size $p$, said PCI system comprising an X-ray source positioned at a distance $Z_{SG}$ from a pre-sample mask, **characterized in that** the lateral displacement error is calculated and corrected by the steps of:

- extracting an intensity profile from said adjustment image that corresponds to a signal profile of a single line of pixels of said digital image detector, said single line of pixels being oriented substantially perpendicularly to the direction of the orientation of the apertures of said pre-sample mask of said PCI system,
- calculate a rolling binning value $I$ of at least first 8 pixels from single pixel values $pv_k$ as;

$$I_k = pv_k + pv_{k+1} \quad ;$$

$k$ being the order number of a pixel in said intensity profile;
- calculating the lateral deviation $\mu$ from the optimal lateral position as;

$$\mu = \frac{s_p}{2} . \frac{D - C}{D + C} \quad ,$$

wherein

$$C = -2\ln\left(\frac{I_1}{I_2}\right); D = -2\ln\left(\frac{I_3}{I_2}\right);$$

and $s_p$ is a pixel size of the digital image detector,
- applying the calculated lateral deviation $\mu$ to said pre-sample mask to perform said lateral deviation adjustment.

6. Method to optimize the alignment of a pre-sample mask in a PCI system according to Claim 1, said PCI system comprising a digital image detector positioned at a distance $Z_{GD}$ from said pre-sample mask of which a detector grating is oriented substantially in parallel with the apertures of said pre-sample mask, **characterized in that** in a preparation step, a lookup table is created of $LT$ values for a number $i$ of known lateral deviations $\mu_i$ between said pre-sample mask and detector grating, by calculating and storing a value $LT_i$ for each of said known lateral deviations $\mu_i$, said value of $LT_i$ being calculated by:

1) extracting for each of said known lateral deviations $\mu_i$ an intensity profile from an adjustment image that was acquired when said system was subjected to said known positional difference $\mu_i$, said intensity profile corresponding to a single line of pixels of said digital image detector, said single line of pixels being oriented perpendicularly to the direction of the orientation of the apertures of said pre-sample mask of said PCI system,
2) calculating a rolling binning value I of at least first $N^*$ pixels from the single pixel value $pv$ as;

$$I_k = \mathrm{pv}_k + \mathrm{pv}_{k+1} \quad ,$$

where k is the order number of a pixel,
and calculating

$$LT_i = \frac{I_n - I_{n+2}}{I_{n+1} + I_{n+3}} \quad ,$$

, wherein $n$ is an integer which defines the reference pixel which corresponds to a position difference of 0,
, and **characterized in that** the lateral displacement error is calculated and corrected by the steps of:

- extracting an intensity profile from said adjustment image that corresponds to a signal profile of a single line of pixels of said digital image detector, said single line of pixels being oriented substantially perpendicularly to the direction of the orientation of the apertures of said pre-sample mask of said PCI system,
- calculating a rolling binning value $I$ of at least first 8 pixels from single pixel values $pv_k$ as;

$$I_k = pv_k + pv_{k+1} \;;$$

$k$ being the order number of a pixel in said intensity profile;

- calculating

$$ref = \frac{I_n - I_{n+2}}{I_{n+1} + I_{n+3}}$$

- comparing and interpolating $ref$ against said stored $LT_i$ values, to obtain a lateral deviation $\mu$ for said alignment,
- applying said lateral deviation $\mu$ to said pre-sample mask to perform said lateral deviation adjustment

**7.** Method to optimize the alignment of a pre-sample mask in a PCI system according to Claim 1, Claim 3 and Claim 4, said PCI system comprising an X-ray source positioned at a distance $Z_{SG}$ from a pre-sample mask, **characterized in that** the tilt angle of the pre-sample mask with respect to a plane that is substantially perpendicular to the line between the X-ray source and the centre of the image detector is calculated and corrected by the steps of:

- calculating magnification errors $M_1$, $M_2$ in at least 2 different areas of the detector located at a distance $l$ from each other,
- calculating said tilt angle in the direction from $M_1$ to $M_2$ as;

$$\beta = \arctan\left(\frac{(M_1 - M_2) \cdot Z_{SG}}{l}\right)$$

- applying the tilt correction $\beta$ to said pre-sample mask to perform said tilt adjustment.

**8.** Method to optimize the alignment of a pre-sample mask in a PCI system according to Claim 6, said PCI system comprising an X-ray source positioned at a distance $Z_{SG}$ from a pre-sample mask, **characterized in that** the at least 2 different areas of the detector are preferably located at opposite edges of the detector and mirrored around X- or Y-axis for which respective tilt angles $\beta_x$ or $\beta_y$ are calculated as;

$$\beta_x = \arctan\left(\frac{(M_{1x} - M_{2x}) \cdot Z_{SG}}{l}\right) \quad ,$$

or respectively;

$$\beta_y = \arctan\left(\frac{(M_{1y}{}' - M_{2y}) \cdot Z_{SG}}{l}\right)$$

9. Method according to Claim 2, wherein the angle $\alpha$ is applied as a digital rotation to the adjustment image before performing further adjustment steps (ii) to (iv).

10. Method for adjusting the position of a pre-sample mask in a PCI system, comprising the adjustment steps of any of the previous claims and wherein a single adjustment image is used for the different adjustment steps.

11. Method according to any of the previous claims wherein the adjustment image is normalized after the acquisition.

12. Method according to any of the previous claims wherein shading correction is applied to the single adjustment image after the acquisition.

13. Method according to any of the previous claims wherein the detector grating is a virtual grating.

Fig. 1

Fig. 2a

Fig. 2b

Fig. 3

column 35                    column 26

Fig. 4

Fig. 5

Look-up table

Fig. 6

Fig. 7a                    Fig. 7b                         Fig. 7c

Fig. 8a

Fig. 8b

**EP 3 570 015 A1**

Europäisches
Patentamt
European
Patent Office
Office européen
des brevets

**EUROPEAN SEARCH REPORT**

Application Number

EP 18 17 3143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | T. P. MILLARD ET AL: "Method for automatization of the alignment of a laboratory based x-ray phase contrast edge illumination system", REVIEW OF SCIENTIFIC INSTRUMENTS, vol. 84, no. 8, 2013, page 083702, XP055077086, ISSN: 0034-6748, DOI: 10.1063/1.4816827 * figures 1,6 * * paragraph [00IV] - paragraph [000V] * | 1-13 | INV. G01N23/041 A61B6/00 |
| A | KREJCI FRANTISEK ET AL: "Hard x-ray phase contrast imaging using single absorption grating and hybrid semiconductor pixel detector", REVIEW OF SCIENTIFIC INSTRUMENTS, AIP, MELVILLE, NY, US, vol. 81, no. 11, 5 November 2010 (2010-11-05), pages 113702-113702, XP012145818, ISSN: 0034-6748, DOI: 10.1063/1.3499372 * figures 1,4,7 * * paragraph [IIIA.] * | 1-13 | |
| A | KALLON G K ET AL: "Comparing signal intensity and refraction sensitivity of double and single mask edge illumination lab-based x-ray phase contrast imaging set-ups", JOURNAL OF PHYSICS D: APPLIED PHYSICS, INSTITUTE OF PHYSICS PUBLISHING LTD, GB, vol. 50, no. 41, 18 September 2017 (2017-09-18), page 415401, XP020320480, ISSN: 0022-3727, DOI: 10.1088/1361-6463/AA8692 [retrieved on 2017-09-18] * paragraph [2.1.]; figures 1,2 * | 1-13 | TECHNICAL FIELDS SEARCHED (IPC) G21K G01N A61B |

-/--

The present search report has been drawn up for all claims

| Place of search | Date of completion of the search | Examiner |
|---|---|---|
| The Hague | 30 November 2018 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

21

Europäisches
Patentamt

European
Patent Office

Office européen
des brevets

## EUROPEAN SEARCH REPORT

Application Number

EP 18 17 3143

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2014/027333 A1 (KONINKL PHILIPS NV [NL]; PHILIPS DEUTSCHLAND GMBH [DE]) 20 February 2014 (2014-02-20) * page 4, line 30 - page 6, line 6 * ----- | 1-13 | |
| A | US 2016/038107 A1 (BATURIN PAVLO [US] ET AL) 11 February 2016 (2016-02-11) * paragraph [0003] * * paragraph [0008] * * paragraph [0032] - paragraph [0035] * ----- | 1-13 | |

TECHNICAL FIELDS
SEARCHED      (IPC)

The present search report has been drawn up for all claims

| Place of search | Date of completion of the search | Examiner |
|---|---|---|
| The Hague | 30 November 2018 | Baranski, Jörg |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 17 3143

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2018

| Patent document cited in search report | Publication date | Patent family member(s) | Publication date |
|---|---|---|---|
| WO 2014027333 A1 | 20-02-2014 | NONE | |
| US 2016038107 A1 | 11-02-2016 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7869567 B

- US 9171650 B

**Non-patent literature cited in the description**

- **PETER R.T. MUNRO ; KONSTANTIN IGNATYEV ; ROBERT D. SPELLER ; ALESSANDRO OLIVO.** *OPTICS EXPRESS,* 01 March 2010, vol. 18 (5), 4103